# EUROPÄISCHE PATENTSCHRIFT

(11) **EP 2 049 449 B1**
(45) Veröffentlichungstag und Bekanntmachung des Hinweises auf die Patenterteilung: **18.07.2018**
(21) Anmeldenummer: 07788363.5
(22) Anmeldetag: 10.08.2007
(51) Int. Cl.: C04B 35/14, C04B 35/46, C04B 35/447, C04B 38/00

(54) **PORÖSER FESTKÖRPER MIT BIMODALER PORENGRÖSSENVERTEILUNG SOWIE VERFAHREN ZU DESSEN HERSTELLUNG**
A POROUS SOLID BODY WITH BIMODAL PORE SIZE DISTRIBUTION AND A METHOD FOR THE PRODUCTION THEREOF
CORPS SOLIDE POREUX AVEC DISTRIBUTION BIMODALE DE LA TAILLE DES PORES ET PROCÉDÉ POUR SA PRÉPARATION

(30) Priorität: 10.08.2006 DE 102006037497
(43) Veröffentlichungstag der Anmeldung: 22.04.2009
(73) Patentinhaber: Biocer-Entwicklungs-GmbH, 95447 Bayreuth (DE)
(72) Erfinder: DITTERT, Bernd, A-4400 Steyer (AT); ZIEGLER, Günter, 90451 Nürnberg (DE); HEIDENAU, Frank, 91257 Pegnitz (DE)
(74) Vertreter: Isarpatent
(86) Internationale Anmeldenummer: PCT/EP2007/058314
(87) Internationale Veröffentlichungsnummer: WO 2008/017719

(56) Entgegenhaltungen:
- WO-A-03/027348
- WO-A-03/062163
- WO-A-2004/026346
- WO-A-2004/103421
- WO-A-2004/112855
- DE-A1- 19 825 419
- US-A1- 2007 110 824
- PADILLA S ET AL: "Processing and mechanical properties of hydroxyapatite pieces obtained by the gelcasting method" JOURNAL OF THE EUROPEAN CERAMIC SOCIETY, ELSEVIER SCIENCE PUBLISHERS, BARKING, ESSEX, GB, Bd. 25, Nr. 4, April 2005 (2005-04), Seiten 375-383, XP004685809 ISSN: 0955-2219

## Beschreibung

Die vorliegende Erfindung betrifft einen porösen Festkörper mit bimodaler Porengrößenverteilung, ein aus diesem Festkörper gewonnenes Granulat, einen Formkörper umfassend den Festkörper oder das Granulat, die Verwendung des Festkörpers, des Granulats oder des Formkörpers, ein Verfahren zur Herstellung des Formkörpers, ein Verfahren zur Herstellung des porösen Festkörpers sowie einen Körper, der mit dem porösen Festkörper beschichtet ist.

Bei Knochendefekten werden in zunehmendem Maße synthetische Knochenersatzmaterialien als Ersatz für den natürlichen Knochen verwendet. Dabei werden in wachsendem Umfang Materialien auf der Basis von Calciumphosphat und Siliciumdioxid in verschiedenen Zusammensetzungen verwendet. Derartige Festkörper verbinden die Bioabbaubarkeit bzw. Biodegradierbarkeit von Calciumphosphatmaterialien mit der Osteoinduktivität von Biogläsern. Granulate und Formkörper auf dieser Basis dienen dem Auffüllen größerer Knochendefekte sowohl im lasttragenden Skelett- als auch im Kieferbereich.

Aus der WO 2004/103421 ist beispielsweise ein Granulat bekannt, das Calciumphosphat in einer Siliciumdioxid-Xerogel-Matrix eingebettet umfasst, wobei das Calciumphosphat frisch aus einer Lösung ausgefällt werden muss und anschließend direkt zu dem Granulat umgesetzt wird. Dabei birgt dieses Herstellungsverfahren den Nachteil, dass die Herstellung lediglich durch frisch gefälltes Hydroxylapatit möglich ist. Eine Verarbeitung von trockenen Kristallen aus beispielsweise Hydroxylapatit kann nicht erfolgen.

Bei operativen Eingriffen besteht das Risiko, dass infektiöse Keime eingeschleppt werden. Dies ist insbesondere dann der Fall, wenn Fremdkörper wie Implantate oder Knochenersatzmaterialien in den Körper eingebracht werden. Im Laufe einer Implantation kommt es dann zum Wettbewerb zwischen körpereigenen Zellen und den eingebrachten Bakterien um die Belegung der zunächst sterilen Oberfläche eines Knochenersatzmaterials. Eine Erstbelegung der Oberfläche mit Bakterien kann eine Entzündungsreaktion des Körpers auslösen, infolge derer das Implantat abgestoßen wird. Es besteht daher der Bedarf an einem Knochenersatzmaterial, das die Erstbelegung eines derartigen Knochenersatzmaterials mit körperfremden Zellen verhindert. Dies kann erreicht werden, wenn das Material an der Oberfläche Keime abtötet, aber gleichzeitig das Wachstum neuen Knochens nicht hindert oder gar fördert.

Ein antimikrobielles, wasserunlösliches Silicatglaspulver ist beispielsweise aus der WO 2003/062163 bekannt. Ein entsprechendes Borsilikatglas ist aus der WO 2004/076369 bekannt. Bei beiden Gläsern ist jedoch eine sehr hohe Temperatur von über 1600°C nötig, um die Gläser herzustellen. Zudem werden diese Gläser nur langsam von Osteoclasten bzw. Osteoblasten zu körpereigenem Knochen umgewandelt.

Aus der DE 10 2004 012 411 A1 ist ein Kompositmaterial auf der Basis von Polykieselsäuren bekannt, das einen Anteil eines organischen Polymers aufweist. Dieses Material besitzt jedoch den Nachteil, dass es aufgrund der organischen Bestandteile eine verringerte Lagerstabilität aufweist und mit der in der Medizintechnik üblichen Methode der Sterilisation durch γ-Bestrahlung nur schwer sterilisiert werden kann. Eine Nachsterilisation in der Klinik ist ebenfalls erschwert, da ein Erhitzen des Materials auf 132 °C zu einer Degradation der organischen Bestandteile führt.

Die DE 102 43 132 B4 offenbart eine antimikrobielle Titanoxidbeschichtung für Implantate, die mittels eines Sol-Gel-Verfahrens hergestellt wird. Diese Beschichtung besitzt jedoch den Nachteil, dass sie sich nicht als Festkörper ausgestalten lässt und daher nicht als vollständiges Knochenersatzmaterial dienen kann. Eine Integration des Implantats in den bestehenden Knochenapparat oder eine Umwandlung des Implantats in natürlichen Knochen ist durch diese Beschichtung nicht möglich.

Ein Knochenersatzmaterial in Form eines Nanoglaspulvers ist aus der DE 10 2004 026 433 bekannt. Dieses Glas- oder Glaskeramikpulver wird in Form eines Granulats mit einer mittleren Partikelgröße kleiner als 1 µm als Knochenersatzmaterial verwendet. Ein einheitlicher Festkörper, der als Knochenersatz direkt verwendet werden kann, kann jedoch nicht mit einem derartigen Glaspulver erzielt werden.

WO2004/112855 offenbart Knochenaufbaumittel aus porösem Calciumphosphat mit isotroper Sinterstruktur und statistisch zwischen den versinterten Partikeln des Calciumphosphates verteilten Poren in mehreren diskreten Grössenverteilungen.

WO2004/026346 offenbart ein Verfahren zur Herstellung einer Titanoxid-Beschichtung auf einem Implantat mit den Schritten: a) Versetzen einer Zubereitung, enthaltend ein organisches Lösungsmittel und einen metallorganischen Titanoxid-Precursor mit Metallsalzen und/oder mit metallorganischen Verbindungen, um Metallionen homogen in der Zubereitung zu verteilen, b) Aufbringen der unter a) hergestellten Zubereitung auf ein Implantat, c) Trocknen der aufgebrachten Beschichtung.

Der vorliegenden Erfindung liegt daher die Aufgabe zugrunde, ein Knochenersatzmaterial bereitzustellen, auf dessen Oberfläche Keime abgetötet werden und das gleichzeitig gut vom Körper resorbiert wird. Eine weitere Aufgabe der vorliegenden Erfindung ist es, ein einfaches und ökologisch verträgliches Herstellungsverfahren bereitzustellen, bei dem keine schädlichen Lösemittel verwendet werden. Eine weitere Aufgabe der vorliegenden Erfindung ist es, ein Granulat zur einfachen Verarbeitung zu einem Knochenersatzmaterial und einen Formkörper als Knochenersatzmaterial bereitzustellen. Schließlich ist es eine Aufgabe der vorliegenden Erfindung, einen beschichteten Körper bereitzustellen, der als Beschichtung ein Knochenersatzmaterial trägt, dessen Oberfläche Keime abtötet und eine gute Akzeptanz des Implantats im Körper gewährleistet.

Erfindungsgemäß werden diese Aufgaben durch die unabhängigen Ansprüche gelöst. Vorteilhafte Ausgestaltungen finden sich in den abhängigen Ansprüchen.

Gemäß einem ersten Aspekt der vorliegenden Erfindung wird ein poröser Festkörper mit einer bimodalen Porengrößenverteilung bereitgestellt, der Partikel in einer oxidischen Matrix und einen Gehalt an Metall-Kationen, der antimikrobiell wirkt, umfasst, wobei die Partikel ausgewählt sind aus calcium- und/oder phosphorhaltigen Verbindungen, TiO₂, SiO₂, ZrO₂ und Al₂O₃, und wobei die Partikel eine Partikelgröße von 0,2-1000 µm aufweisen, die oxidische Matrix ein oder mehrere Oxide der Elemente Al, Ca, Mg, P, Si, Ti und/oder Zr enthält, und die bimodale Porengrößenverteilung derart ausgestaltet ist, dass Nanoporen im Bereich von 1 bis 100 nm in der oxidischen Matrix vorliegen und der Festkörper des Weiteren Makro-Poren mit einer Größe von 50 bis 1.000 µm aufweist.

Die Metall-Ionen können im Körper an die Umgebung abgegeben werden und somit die antimikrobielle Wirkung erzeugen. Durch die Abgabe der Metall-Kationen werden auf der Oberfläche und in der Umgebung befindliche Keime abgetötet. Durch die bimodale Porengrößenverteilung des Festkörpers kann der Festkörper innerhalb des Organismus leicht in nativen Knochen umgewandelt werden. Die Porengröße ist dabei derart ausgelegt, dass diese Porenstruktur den Aufbau neuen Knochens unterstützt. Die Porengröße im Bereich von 50 bis 1.000 µm unterstützt das Eindringen neuer Knochenzellen in den porösen Festkörper, der dadurch leichter in neues Knochenmaterial umgewandelt werden kann.

Gemäß einer bevorzugten Weiterbildung der vorliegenden Erfindung besteht die oxidische Matrix aus einem oder mehreren der Oxide Al₂O₃, TiO₂, SiO₂ und ZrO₂. Geeignet sind jedoch alle Oxide, und insbesondere die Oxide der Elemente Al, Ca, Mg, P, Si, Ti und Zr, die sich zu einer Sol-Gel-Verarbeitung eignen. Insbesondere bevorzugt besteht die Matrix aus Tricalciumphosphat, Hydroxylapatit, Al₂O₃, TiO₂, SiO₂ oder ZrO₂.

Die oxidische Matrix kann in einem beliebigen Verhältnis zu den eingebrachten Partikeln vorliegen, solange die Matrix die Partikel zu einem Festkörper verbinden kann. Bevorzugt beträgt der Anteil der oxidischen Matrix an dem Festkörper von 20 bis 99 Gew.-%, bevorzugter von 30 bis 70 Gew.-%.

In diese oxidische Matrix werden gemäß der Erfindung Partikel eingebracht. Diese Partikel umfassen zumindest anorganische Partikel und sind ausgewählt aus einer phosphorhaltigen Verbindung, insbesondere einer Phosphatverbindung, TiO₂, SiO₂, ZrO₂ und Al₂O₃. Bevorzugte calcium- und/oder phosphorhaltige Verbindungen sind Calciumphosphate, insbesondere bevorzugt Hydroxylapatit und/oder β-Tricalciumphosphat. Die Partikel können dabei aus dem gleichen Material wie die oxidische Matrix aufgebaut sein, oder aus einem unterschiedlichen Material bestehen. Bevorzugt bestehen die Partikel aus einem anderen Material als die oxidische Matrix. Wird ein Calciumphosphat als Partikel verwendet, kann die Resorption des Knochenersatzmaterials durch den Körper besonders gut erfolgen. Es können auch Partikel unterschiedlicher Materialien in die Matrix eingebracht werden. Dabei können die unterschiedlichen Partikel unterschiedliche Funktionen erfüllen.

Die Partikel werden in den porösen Festkörper in einer Partikelgröße von 0,2-1000 µm, bevorzugt mit einer Größe von 0,5-100 µm, und am bevorzugtesten mit einer Größe von 1-10 µm eingebracht. Durch die Auswahl der Partikelgröße in diesem Bereich wird sichergestellt, dass eine bimodale Porengrößenverteilung in dem Festkörper vorliegt. Zum einen werden Poren von der Größenordnung der Partikel zwischen den Partikeln ausgebildet. Zum anderen werden kleinere Poren im Bereich der Größenordnung der Nanopartikel, die zum Erzeugen der Sol-Gel-Matrix verwendet werden, erzeugt. Daher liegen im porösen Festkörper gemäß der vorliegenden Erfindung einerseits Poren im Nanometerbereich und andererseits Poren im Micrometerbereich vor. Durch diese Porenverteilung wird eine schnelle Umwandlung des Materials zu natürlichem, körpereigenen Knochen begünstigt. Der Begriff "bimodale Porengrößenverteilung" bedeutet hierbei, dass in einem Diagramm, in welchem die Häufigkeit von Poren oder das spezifische Porenvolumen oder die spezifische Porenoberfläche über der Porengröße aufgetragen sind, zwei Maxima der Verteilungskurve vorliegen. Das eine Maximum liegt dabei im Nanometer-Bereich, das andere im MicrometerBereich.

Der poröse Festkörper der vorliegenden Erfindung weist einen Gehalt an Metall-Kationen auf, der antimikrobiell wirkt. Als Metall-Kationen können beispielsweise Ionen der Metalle Kupfer, Silber, Chrom, Zink, Kobalt, Vanadium und Quecksilber, insbesondere Cu⁺, Cu²⁺, Ag⁺, Ag²⁺, Cr³⁺, Cr⁶⁺, Zn²⁺, Co²⁺ , V⁵⁺ und Hg⁺ verwendet werden. Bevorzugt werden die Metall-Kationen mit antimikrobieller Wirkung in Form eines Salzes eingebracht, wobei das Anion dieses Salzes biokompatibel ist. Besonders bevorzugt werden als Anionen organische Anionen verwendet. Beispielsweise können die Metalle in Form ihrer Acetate, insbesondere Kupfer- und/oder Silberacetat, in den porösen Festkörper eingebracht werden. Werden biokompatible Salze der Metallkationen verwendet, so können die Anionen der Salze im Körper ohne Nebenwirkungen abgebaut werden. Die Anionen stören somit die Resorption des Materials nicht. In einer besonders bevorzugten Weiterbildung der vorliegenden Erfindung sind die verwendeten Metallsalze lösliche Metallsalze, bevorzugt in Wasser lösliche Metallsalze.

Neben den Metall-Ionen mit antimikrobieller Wirkung können zusätzlich weitere Metall-Ionen in der oxidischen Matrix und/oder den Partikeln des porösen Festkörpers enthalten sein, die weitere medizinisch-pharmazeutische Wirkungen aufweisen. Bevorzugt werden hier Zn²⁺ und Ca²⁺ Ionen in definierter Konzentration zur Förderung des Knochenwachstums in den porösen Festkörper eingebracht. Durch die Einstellung der Konzentration der knochenwachstumsfördernden Metallkationen kann die Geschwindigkeit der Umwandlung des Knochenersatzmaterials in körpereigenen Knochen gezielt gesteuert werden. Durch die Einstellung eines Konzentrationsgradienten beispielsweise in den Partikeln kann das Knochenwachstum gezielt im Verlauf der Zeit gesteuert werden.

Gemäß einer bevorzugten Weiterbildung des ersten Aspektes der vorliegenden Erfindung weist der poröse Festkörper eine bimodale Porengrößenverteilung in der Form auf, dass der Festkörper einerseits Poren im Nanometerbereich, so genannte Mesoporen, im Bereich von 1-100 nm, bevorzugt im Bereich von 2-50 nm, bevorzugter im Bereich von 5-30 nm und am bevorzugtesten im Bereich von 7-15 nm in der oxidischen Matrix aufweist. Der Festkörper weist zum anderen Poren im Mikrometerbereich, mit einer Größe von 50-1000 µm, bevorzugt 100-1000 µm, weiter bevorzugt 200-500 µm und am bevorzugtesten von 250-350 µm auf. Die Poren im Nanometerbereich werden dabei in der oxidischen Matrix zwischen den Sol-Gel-Partikeln der Matrix ausgebildet. Die Poren im Mikrometerbereich bilden sich zwischen den im Festkörper vorhandenen Partikeln des erfindungsgemäßen Festkörpers aus.

Die Dichte des porösen, anorganischen Festkörpers liegt bevorzugt im Bereich von 0,9 - 2,0 g/cm³, bevorzugter im Bereich von 1,0 - 1,5 g/cm³ und am bevorzugtesten im Bereich von 1,1 - 1,3 g/cm³. Die Porosität des Festkörpers liegt entsprechend bevorzugt bei 30-80 %, weiter bevorzugt bei 35-70 % und am bevorzugtesten zwischen 40 und 60 %.

Die Gel-Matrix des Festkörpers gemäß der vorliegenden Erfindung kann teilweise oder vollständig in eine Glasmatrix umgewandelt werden. Dabei können zusätzlich Netzwerkwandleroxide, wie beispielsweise Na₂O, in den porösen Festkörper eingebracht werden. Bei einer Erwärmung im Bereich von 700-1200°C, bevorzugt von 800-1000°C wird die gelartige Matrix des porösen Festkörpers in eine Glasmatrix umgewandelt. Dadurch kann die Festigkeit des porösen Festkörpers weiter erhöht werden. Die Resorption eines derartigen Festkörpers kann auch über den Anteil der Glasmatrix in dem Festkörper gesteuert werden. Je höher der Anteil glasartiger Bereiche in dem Festkörper ist, desto langsamer wird der Festkörper resorbiert.

Die Resorptionsgeschwindigkeit des Festkörpers kann auf unterschiedliche Arten gesteuert werden. So trägt beispielsweise der Anteil glasartiger Bereiche ebenso zur Resorptionsgeschwindigkeit bei wie der Anteil weiterer Metalle wie Zink oder Calcium. Außerdem kann auch die Porengrößenverteilung die Resorptionsgeschwindigkeit beeinflussen.

Ein poröser Festkörper gemäß der vorliegenden Erfindung kann Partikel aus TiO₂ umfassen. Der Festkörper kann neben Partikeln aus TiO₂ auch andere Partikel enthalten. Es ist jedoch auch möglich, dass lediglich Partikel aus TiO₂ vorliegen. Ein derartiger Festkörper, der Partikel aus TiO₂ umfasst, kann unter UV-Strahlung photokatalytisch Keime abtöten. Das Material kann somit auch als Filter verwendet werden, durch den beispielsweise Wasser unter UV-Bestrahlung hindurchströmt und innerhalb dieses Filters über die photokatalytische Wirkung der mit UV-Strahlen bestrahlten TiO₂-Partikel Keime abgetötet werden. Der Festkörper besitzt somit zwei unterschiedliche, antimikrobielle Bereiche. Zum einen werden Keime durch die antimikrobiellen Metallkationen abgetötet, zum anderen werden durch die über UV-Strahlung aktivierten TiO₂-Partikel Keime abgetötet.

Des Weiteren lässt sich die Röntgenopazität des porösen Festkörpers über einen Gehalt an Metall-Kationen einstellen. Das röntgenopake Verhalten von Knochenersatzmaterialien ist beispielsweise wichtig bei der späteren Kontrolle der Knochenersatzmaterialien. Die Opazität des Materials kann beispielsweise gegenüber natürlichem Knochen erhöht sein. Somit lassen sich der Abbau des Knochenersatzmaterials und die Umwandlung zu natürlichem Knochen überwachen. Zum anderen kann die Opazität des Materials auch derart eingestellt sein, dass es sich von umgebenden Weichteilen in einer Röntgenaufnahme abgrenzt.

Als Ionen zur Einstellung der Röntgenopazität können bevorzugt schwere Ionen eingebracht werden. Es können jedoch auch die Konzentrationen leichter Metall-Kationen wie beispielweise Calcium oder auch der obengenannten, antimikrobiell wirkenden MetallKationen derart erhöht werden, dass die Durchlässigkeit des derart hergestellten Materials für Röntgenstrahlung abnimmt. Der Kontrast des porösen Festkörpers gemäß der vorliegenden Erfindung kann somit gegenüber dem umgebenden Weichteilgewebe erhöht werden. Insbesondere bevorzugt wird die Röntgenopazität über Zirkonatome eingestellt, da schwere Atome einen größeren Einfangquerschnitt für Röntgenstrahlen besitzen.

Ein zweiter Aspekt der vorliegenden Erfindung betrifft ein Granulat aus einem porösen Festkörper. Bevorzugt besitzt dabei das Granulat eine Partikelgröße, die die Porengröße der größeren Poren in der bimodalen Porengrößenverteilung um mindestens das Fünffache übersteigt. Damit ist die Partikelgröße des Granulats mindestens fünfmal so groß wie die größte Pore des Festkörpers. Dadurch kann im Granulat die Wirkung der bimodalen Porengrößenverteilung des Festkörpers der vorliegenden Erfindung seine Wirkung voll entfalten.

Ein dritter Aspekt der vorliegenden Erfindung betrifft einen Formkörper oder eine Beschichtung, die neben einem erfindungsgemäßen Festkörper, der auch in Form eines Granulats vorliegen kann, eine ausgehärtete Flüssigkeit umfassen. Diese ausgehärtete Flüssigkeit ist bevorzugt biologisch abbaubar. Insbesondere bevorzugt wird ein Polylactid oder Polysaccharid verwendet. Beim biologischen Abbau von Polylactid entsteht eine Säure, die den Abbau des Formkörpers beschleunigt und durch die Freisetzung von Calciumionen das Knochenwachstum beschleunigt. Zudem lässt sich der Formkörper durch das Gießen in eine Form und die anschließende Bearbeitung des Formkörpers mittels herkömmlicher Verfahren in eine gewünschte Form bringen.

Ein weiterer Aspekt der vorliegenden Erfindung betrifft die Herstellung eines oben genannten Formkörpers oder einer Beschichtung. Dabei wird ein Festkörper und/oder Granulat in eine aushärtbare Flüssigkeit eingebracht, um eine Mischung herzustellen, die anschließend in eine Form gebracht bzw. als Beschichtung appliziert wird. Die Form kann dabei eine endkonturnahe Form sein, in die das Material gegossen wird. Durch Aushärten der aushärtbaren Flüssigkeit entsteht ein Formkörper bzw. eine Beschichtung. Das Aushärten kann dabei beispielsweise durch Erwärmen oder auch durch Bestrahlen erfolgen.

Ein fünfter Aspekt der vorliegenden Erfindung betrifft die Herstellung eines porösen Festkörpers mit den Schritten:
(a) Bereitstellen seines Sols in Form einer weitestgehend monodispersen Suspension aus Nanopartikeln mindestens eines Oxids der Elemente Al, Ca. Mg, P, Si, Ti und Zr, wobei die Nanopartikel eine Partikelgröße im Bereich von 0,1-50 nm aufweisen;
(b) Einbringen von Partikeln in die Suspension, wobei die Partikel ausgewählt sind aus calcium- und/oder phosphorhaltigen Verbindungen, TiO₂, SiO₂, ZiO₂ und Al₂O₃, wobei die Partikel eine Partikelgröße von 0,2-1000 µm aufweisen;
(c) Einbringen eines Metallkationen enthaltenden Salzes, wobei die Metallkationen antimikrobielle Wirkung besitzen;
(d) Überführen des Sols in ein Gel;
(e) Trocknen des Gels, um einen porösen Festkörper zu erzeugen.

Das Sol kann kommerziell erworben werden oder aus den Einzelkomponenten hergestellt werden. Das kommerziell erworbene Sol liegt bevorzugt in Form einer weitestgehend monodispersen Suspension aus Nanopartikeln eines Oxids vorliegen.

Zur Herstellung eines Sols in Form einer weitestgehend monodispersen Suspension aus Nanopartikeln eines Oxids werden Nanopartikel eines Oxids in ein Lösemittel eingebracht. Als Lösemittel dient hier bevorzugt Wasser. Durch Einstellen des pH-Wertes kann die Umwandlung des Sols in ein Gel erfolgen. Vor der Umwandlung des Sols in ein Gel werden sowohl das metallhaltige Salz als auch die Partikel in die Lösung eingebracht. Die Partikel werden in der Lösung dispergiert, sodass eine Suspension entsteht. Die Salze eines antimikrobiell wirkenden Metall-Kations werden bevorzugt in dem Sol gelöst. Bei der Überführung des Sols in ein Gel kann das Gel anschließend in eine Form gegossen werden. Nach Trocknen des Gels entsteht ein poröser Festkörper in Form einer Matrix, die bevorzugt gelartig ausgestaltet ist. Mittels dieses Verfahrens kann ein Festkörper in im Verhältnis zum Stand der Technik kurzen Zeiträumen von beispielsweise weniger als einer Stunde aus dem Sol hergestellt werden. Dieser Grünkörper kann bei geringen Temperaturen, beispielsweise im Bereich von Raumtemperatur (20°C) bis 110°C, bevorzugt im Bereich von Raumtemperatur bis 80°C, innerhalb weniger Stunden, beispielsweise innerhalb von bis zu 5 Stunden, bevorzugt bis zu 3 Stunden, getrocknet werden, um den porösen, anorganischen Festkörper gemäß der Erfindung herzustellen. Das Verfahren ist somit gegenüber den meisten Verfahren des Standes der Technik, die einen langwierigen Sol-Gel-Schritt und ein Brennen des Grünkörpers benötigen, sehr ökonomisch in Bezug auf die Zeit und die benötigte Energie.

Bei der Verwendung von Wasser als Lösemittel besitzt das Verfahren den Vorteil, dass kein meist als schädlich betrachtetes organisches Lösemittel an dem Verfahren beteiligt ist, das möglicherweise nach Herstellen des Festkörpers entfernt werden muss.

Bevorzugt wird das Sol als monodisperse Suspension von Nanopartikeln verwendet. Diese Suspension kann in-situ hergestellt werden oder als solche bereitgestellt werden. Bei dem erfindungsgemäßen Verfahren ist es wichtig, dass die anorganischen Partikel in eine Sol-Suspension, bevorzugt eine monodisperse Sol-Suspension, eingebracht werden, in der bevorzugt auch das Metallsalz löslich ist. Das eingesetzte Sol ist ein bevorzugt ein partikuläres Sol und kein polymeres Sol. Unter einem polymeren Sol wird dabei ein Sol verstanden, das aus Vorläufermaterialien in-situ hergestellt wird. Das partikuläre Sol ist bevorzugt ein Sol, das eine Dispersion von Nanopartikeln, bevorzugt mit einer Partikelgröße im Bereich von 0,1-50 nm, weiter bevorzugt im Bereich von 1-30nm, am bevorzugtesten im Bereich von 7-20 nm, ist. Es wird angenommen, ohne jedoch darauf festgelegt sein zu wollen, dass durch das Einbringen der Partikel in eine bereits vorliegende Suspension eines Sol größere Agglomerate aus Partikeln entstehen, die durch Sol-Nanopartikel zusammen gehalten werden. Es wird weiter angenommen, dass diesen Agglomerate beim Übergang des Sols in ein Gel zum Ausbilden der Makro-Poren eine zusätzliche Rolle zukommt.

Der derart hergestellte poröse Festkörper kann nach dem Trocknen in Schritt (e) optional calciniert werden. Dies dient zur Erhöhung der Festigkeit. Bei höheren Temperaturen kann die Bildung von Glasphasen erfolgen. Über die Art der Glasphase, insbesondere deren Ausbildungsgrad, kann Freisetzung von Ca-Ionen und/oder Metall-Ionen eingestellt werden.

Das Trocknen des Gels erfolgt bevorzugt bei einer Temperatur im Bereich von Raumtemperatur bis 200°C, bevorzugter im Bereich von 50°C bis 150°C, weiter bevorzugt im Bereich von 50 bis 110°C und insbesondere bevorzugt im Bereich von 50 bis 80°C. Bevorzugt wird das Gel an der Luft getrocknet. Am bevorzugtesten wird das Gel bei Raumtemperatur an der Luft getrocknet.

Gemäß einer bevorzugten Ausgestaltung des Verfahrens zur Herstellung eines porösen Festkörpers werden Nanopartikel mit einer Größe von 1 bis 20 nm, bevorzugt mit einer Größe von 2 bis 10 nm und insbesondere bevorzugt mit einer Größe von 5 bis 8 nm zur Herstellung eines Sols verwendet. Insbesondere bevorzugt wird eine wässrige Kieselsäuresuspension oder eine Suspension von Al₂O₃, TiO₂ und/oder ZrO₂ verwendet. Es können jedoch auch andere Oxide oder Mischungen verschiedener Oxide zu einer Suspension verarbeitet werden.

Die Gelbildung aus dem Sol erfolgt bevorzugt durch Ansäuern der monodispersen Suspension, bevorzugt auf einem pH-Wert von 1 bis 9, bevorzugter von 6 bis 8 und am bevorzugtesten im neutralen Bereich von etwa 7. Die Suspension der Nanopartikel des Oxids reagiert basisch. Das Ansäuern kann mit einer beliebigen Säure erfolgen, bevorzugt einer Mineralsäure und insbesondere bevorzugt mit Salpetersäure oder Salzsäure. Der pH-Wert ist dabei derart einzustellen, dass die Gelbildung des Oxids erfolgt.

Nach dem Trocknen des Festkörpers kann dieser auf eine Temperatur von 300°C bis 600°C, bevorzugt von 400°C bis 500°C, erhitzt werden, um organische Rückstände aus dem Festkörper zu entfernen und/oder die gelartige Matrix zu sintern. Organische Rückstände können beispielsweise Polymerpartikel sein, die als Template für Poren dienen, oder Reste organischer Lösemittel oder organischer Liganden der Vorläufermaterialien der Sol-Herstellung sind. Durch das Entfernen der organischen Rückstände wird ein vollständig anorganischer, poröser Festkörper erzeugt. Beim Sintern werden sog. "Sinterhälse" zwischen den Partikeln ausgebildet, die zur Verfestigung des Materials beitragen. Durch das Sintern kann die Festigkeit des Materials weiter erhöht werden.

Gemäß einer anderen bevorzugten Weiterbildung der vorliegenden Erfindung werden zusätzlich zu den anorganischen Partikeln, wie sie oben beschrieben sind, organische Partikel aus einem Polymermaterial in die oxidische Matrix eingebracht. Diese Materialien können beispielsweise Polysaccharide, Polylactide und/oder Polymethylmethacrylat umfassen. Die organischen Partikel aus einem Polymermaterial dienen als Schablone bzw. Platzhalter innerhalb des porösen Festkörpers und werden anschließend bei einer erhöhten Temperatur entfernt, um einen erfindungsgemäßen porösen, anorganischen Festkörper herzustellen. Das Entfernen erfolgt beispielsweise durch Ausbrennen, wobei die organischen Partikel bevorzugt rückstandslos verbrennen.

Der somit vorliegende, vollständig anorganische Festkörper ist sehr lagerstabil und kann durch übliche Verfahren sterilisiert werden. Das Erhitzen auf Temperaturen, wie sie beim Sterilisieren üblich sind, verändert die Struktur des Festkörpers nicht.

Gemäß einer anderen bevorzugten Weiterbildung der vorliegenden Erfindung kann die gelartige Matrix auch in eine Glasmatrix umgewandelt werden. Dies erfolgt bei einer Temperatur von etwa 700°C bis 1200°C, bevorzugt von 800°C bis 1000°C. Dabei bilden sich schmelzflüssige Bereiche innerhalb der gelartigen Matrix aus. Die Partikelstruktur verschwindet zugunsten eines kompakten Glaskörpers. Dieser Glaskörper besitzt eine erhöhte Festigkeit gegenüber dem porösen Festkörper und weist zudem eine verlangsamte Resorption auf.

Gemäß eines weiteren Aspekts der vorliegenden Erfindung kann ein Implantat mit einer Beschichtung versehen werden, die einen porösen Festkörper enthält. Durch die Beschichtung eines Implantats mit einem porösen Festkörper gemäß der vorliegenden Erfindung kann die Integration eines Implantats in einen bestehenden Knochenverbund erleichtert erfolgen, wobei die positiven Eigenschaften der antimikrobiellen Wirkung der Beschichtung eine Abstoßung des Implantats verhindern. Insbesondere kann durch die Beschichtung eine Oberflächenrauheit erreicht werden, die eine optimale Integration des Implantats ermöglicht.

Gemäß eines letzten Aspekts der vorliegenden Erfindung kann der poröse Festkörper oder das Granulat oder der Formkörper gemäß der Erfindung als Knochenersatzmaterial verwendet werden. Der Festkörper oder der Formkörper kann dabei direkt zu einer gewünschten Form verarbeitet werden. Das Granulat kann beim Auffüllen von Knochendefekten oder auch in einer weiteren Matrix als Knochenersatzmaterial verwendet werden.

Im Folgenden wird die Erfindung anhand von bevorzugten Ausführungsbeispielen und Figuren näher erläutert. Die Beispiele sollen dabei jedoch den Schutzbereich des Patentes nicht beschränken und dienen lediglich zum besseren Verständnis der Erfindung.

In den Figuren zeigen
- Figur 1: einen porösen Festkörper aus SiO₂ und Calciumphosphatmaterial ohne Wärmebehandlung;
- Figur 2: das unter Figur 1 dargestellte Material nach einer Wärmebehandlung bei 500°C;
- Figur 3: das unter Figur 1 dargestellte Material nach einer Wärmebehandlung bei 800°C; und
- Figur 4: das unter Figur 1 dargestellte Material nach einer Wärmebehandlung bei 1000°C.

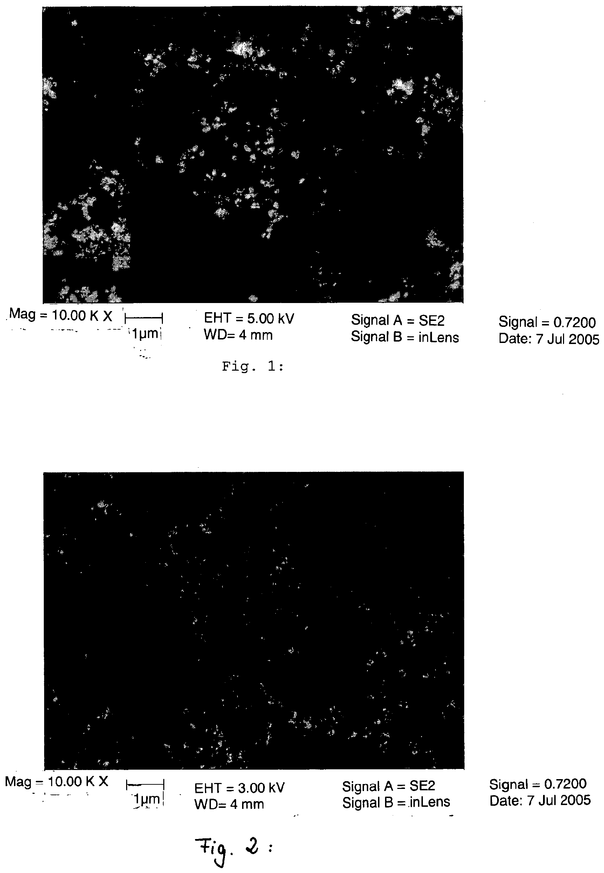

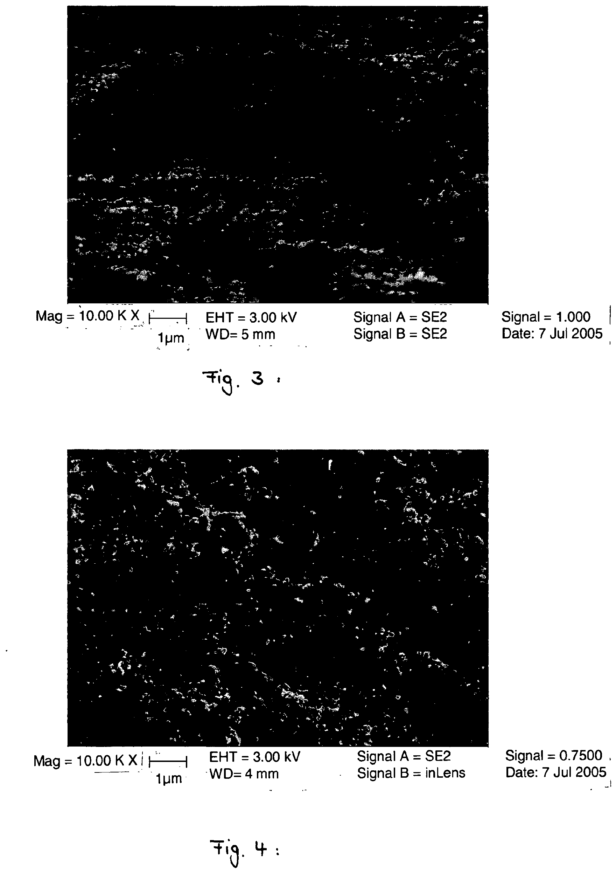

In Figur 1 ist eine Aufnahme eines porösen Festkörpers aus SiO₂ und Calciumphosphatmaterial ohne Wärmebehandlung gezeigt. Die Figuren 2 bis 4 zeigen dieses Material beim Durchlaufen einer Wärmebehandlung nach 500°C, 800°C bzw. 1000°C. Dabei wird die zunehmende Ausbildung glasartiger Bereiche ersichtlich.

### Beispiele

### Ausführungsbeispiel 1:

Ausgangssubstanz für die SiO₂-Gelmatrix ist ein kommerziell erhältliches, wässriges Kieselsol. Dabei handelt es sich um eine Suspension von SiO₂-Partikeln mit einem mittleren Partikeldurchmesser von 8 nm.

Zu diesem wässrigen Sol wird so lange eine verdünnte Salpetersäurelösung zugegeben, bis sich ein pH-Wert von etwa 7 einstellt.

Anschliessend wird ein industriell hergestelltes Hydroxylapatitpulver zugegeben. Das Gewichtsverhältnis Kieselsol: Pulver kann dabei maximal 2 : 1 betragen.

Nach der Dispergierung des Pulvers erfolgt die Zugabe eines Metallcarboxylates, so dass das Gewichtsverhältnis SiO₂ : Metallcarboxylat maximal 48 : 1 beträgt.

Das pulvergefüllte, mit Metall-Ionen (Ag⁺ bzw. Cu²⁺) versetzte Sol wird in zylindrische Teflonformen gegossen. Nach der Gelbildung werden Zylinder mit einer Höhe von etwa 20 mm und einem Durchmesser von etwa 8 mm erhalten. Diese werden bis zur Gewichtskonstanz getrocknet. Nach einer Wärmebehandlung bei 500°C werden aus dem erfindungsgemäßen Festkörper stabile Formkörper mit einer Oberfläche erhalten, die Keime abtötet.

### Ausführungsbeispiel 2:

Das pulvergefüllte, mit Metall-Ionen (Ag⁺ bzw. Cu²⁺) versetzte Sol wird in einem Gefäß beliebiger Geometrie über den erfindungsgemäßen Prozess in ein Gel überführt. Das Gel wird zerkleinert (2-5 mm) und bei 80°C getrocknet. Alternativ wird das Gel an Luft bei Raumtemperatur getrocknet und anschließend zerkleinert. Nach einer Wärmebehandlung bei 500°C wird ein Granulat zum Auffüllen von Knochendefekten in der Human- und Veterinärmedizin mit einer Oberfläche, auf der Keime abgetötet werden, aus dem erfindungsgemäßen Festkörper erhalten.

### Ausführungsbeispiel 3:

Ein erfindungsgemäßer Festkörper oder ein Granulat wird auf 800 bzw. 1000°C aufgeheizt. Mit steigender Temperatur wird so die partikular strukturierte Gelmatrix zunehmend in eine Glasmatrix umgewandelt. Der so erhaltene Festkörper oder das so erhaltene Granulat entspricht einer Glaskeramik mit einer Oberfläche, auf der Keime abgetötet werden.

### Ausführungsbeispiel 4:

In erfindungsgemäß angesäuertes wässriges Kieselsol wird TiO₂-Pulver eingebracht. Das Gewichtsverhältnis Kieselsol: Pulver kann dabei maximal 2 : 1 betragen. Nach der Lufttrocknung bei Raumtemperatur oder alternativ bei 80°C wird ein Granulat erhalten, das, unter UV-Bestrahlung und mit Wasser durchströmt, photokatalytisch Keime im Wasser abtötet.

### Ausführungsbeispiel 5:

In das Sol werden mittels einer Tauchbeschichtungsmaschine Implantate getaucht und anschließend thermisch bei 500°C behandelt. So wird eine Beschichtung erhalten, die eine definierte Oberflächenrauheit besitzt sowie die Eigenschaft, Keime auf ihrer Oberfläche abzutöten.

### Ausführungsbeispiel 6:

Das erfindungsgemäße Granulat wird in eine aushärtende Flüssigkeit, bevorzugt Methylmethacrylat eingebracht. So wird ein Verbundwerkstoff erhalten, bei dem das erfindungsgemäße Granulat in einen Formkörper oder eine Schicht eingelagert ist.

### Ausführungsbeispiel 7:

Das erfindungsgemäße Granulat zeigt aufgrund seines Gehaltes an Metall-Ionen röntgenopakes Verhalten. Daher ist es als Füllstoff für medizinische Füllpräparate (z. B. Knochenzemente) geeignet.

### Ausführungsbeispiel 8:

Calciumphosphat wird in ein wässriges, mit Metallkationen versetztes TiO₂-Sol eingebracht. In das so erhaltene Sol werden Implantate getaucht und anschließend thermisch bei 500°C behandelt. So wird eine Beschichtung erhalten, die eine definierte Oberflächenrauigkeit besitzt sowie die Eigenschaft, Keime auf ihrer Oberfläche abzutöten.

### Ausführungsbeispiel 9:

Das erfindungsgemäße Granulat wird in eine aushärtende Flüssigkeit, bevorzugt Methylmethacrylat eingebracht. Durch eine thermisch induzierte oder eine photoinduzierte Polymerisation wird ein Verbundwerkstoff erhalten, bei dem das erfindungsgemäße Granulat in einen Festkörper oder eine Schicht eingelagert ist. Dieses System dient als Knochenzement, der Keime auf seiner Oberfläche abtötet.

## Patentansprüche

1. Poröser, anorganischer Festkörper mit einer bimodalen Porengrößenverteilung, umfassend Partikel in einer oxidischen Matrix und einen Gehalt an Metallkationen, der antimikrobiell wirkt, wobei
die Partikel ausgewählt sind aus calcium- und/oder phosphorhaltigen Verbindungen, TiO₂, SiO₂, ZrO₂ und Al₂O₃, und wobei die Partikel eine Partikelgröße von 0,2-1000 µm aufweisen,
die oxidische Matrix ein oder mehrere Oxide der Elemente Al, Ca, Mg, P, Si, Ti und Zr enthält, und
die bimodale Porengrößenverteilung derart ausgestaltet ist, dass Nanoporen im Bereich von 1 bis 100 nm in der oxidischen Matrix vorliegen und der Festkörper des Weiteren Makro-Poren mit einer Größe von 50 bis 1.000 µm aufweist.

2. Festkörper nach Anspruch 1, wobei die oxidische Matrix ein oder mehrere Oxide enthält, ausgewählt aus Al₂O₃, TiO₂, SiO₂ und ZrO₂, und/oder wobei der Anteil der oxidischen Matrix in dem porösen Festkörper von 20 bis 99 Gew.-%, bevorzugt von 30 bis 70 Gew.-% beträgt.

3. Festkörper nach Anspruch 1 oder 2, wobei die Partikel ein Calciumphosphat, insbesondere Hydroxylapatit oder β-Tricalciumphosphat, umfassen, und/oder wobei die Partikel eine Partikelgröße von 0,5-100 µm aufweisen.

4. Festkörper nach einem oder mehreren der vorhergehenden Ansprüche, wobei die Metallkationen ausgewählt sind aus Ionen der Metalle Kupfer, Silber, Chrom, Zink, Cobalt, Vanadium und Quecksilber, und insbesondere Cu²⁺, Cu⁺, Ag⁺, Cr³⁺, Cr⁶⁺, Zn²⁺, Co²⁺, V⁵⁺, Hg²⁺ und Hg⁺ sind, und/oder wobei die Metallkationen mit antimikrobieller Wirkung in Form eines Salzes vorliegen, vorzugsweise als Metallsalze mit organischen Anionen, bevorzugt Kupfer- oder Silberacetat.

5. Festkörper nach einem oder mehreren der vorhergehenden Ansprüche, wobei die bimodale Porengrößenverteilung derart ausgestaltet ist, dass Nanoporen im Bereich von 2 bis 50 nm, bevorzugter 5 bis 30 nm und am bevorzugtesten 7 bis 15 nm in der oxidischen Matrix vorliegen, und der Festkörper des weiteren Makro-Poren mit einer Größe von 200 bis 500 µm und bevorzugt 250 bis 350 µm aufweist.

6. Festkörper nach einem oder mehreren der vorhergehenden Ansprüche, wobei die oxidische Matrix und/oder die Partikel zusätzlich weitere Metallionen mit pharmazeutischer Wirkung enthalten, bevorzugt Zn²⁺ und Ca²⁺ zur Förderung des Knochenwachstums, und/oder wobei die oxidische Matrix des Festkörpers teilweise oder vollständig in eine Glasmatrix umgewandelt ist.

7. Granulat aus einem Festkörper gemäß einem der vorhergehenden Ansprüche, wobei das Granulat vorzugsweise eine Partikelgröße besitzt, die die Makro-Porengröße mindestens um das 5-fache übersteigt.

8. Formkörper oder Beschichtung, die neben einem Festkörper gemäß einem oder mehreren der Ansprüche 1 bis 6, bevorzugt in Form eines Granulats gemäß Anspruch 7, eine ausgehärtete Flüssigkeit umfassen, wobei die ausgehärtete Flüssigkeit vorzugsweise biologisch abbaubar ist und bevorzugt ein Polylactid oder Polysaccharid umfasst.

9. Verfahren zur Herstellung eines Formkörpers oder einer Beschichtung gemäß Anspruch 8, wobei ein Festkörper gemäß einem oder mehreren der Ansprüche 1 bis 6, bevorzugt in Form eines Granulats gemäß Anspruch 7, in eine aushärtbare Flüssigkeit eingebracht wird, um eine Mischung herzustellen, anschließend diese Mischung in eine Form gebracht bzw. auf einen Körper aufgebracht wird, und die aushärtbare Flüssigkeit ausgehärtet wird, so dass ein Formkörper bzw. eine Beschichtung entsteht.

10. Verfahren zur Herstellung eines porösen Festkörpers gemäß einem oder mehreren der Ansprüche 1 bis 6 mit den Schritten:
(a) Bereitstellen seines Sols in Form einer weitestgehend monodispersen Suspension aus Nanopartikeln mindestens eines Oxids der Elemente Al, Ca, Mg, P, Si, Ti und Zr, wobei die Nanopartikel eine Partikelgröße im Bereich von 0,1-50 nm aufweisen;
(b) Einbringen von Partikeln in die Suspension, wobei die Partikel ausgewählt sind aus calcium- und/oder phosphorhaltigen Verbindungen, TiO₂, SiO₂, ZrO₂ und Al₂O₃, wobei die Partikel eine Partikelgröße von 0,2-1000 µm aufweisen;
(c) Einbringen eines Metallkationen enthaltenden Salzes, wobei die Metallkationen antimikrobielle Wirkung besitzen;
(d) Überführen des Sols in ein Gel;
(e) Trocknen des Gels, um einen porösen Festkörper zu erzeugen;
wobei das Trocknen in Schritt (e) vorzugsweise bei einer Temperatur im Bereich von Raumtemperatur bis 200°C, bevorzugt von 50°C bis 150°C und besonders bevorzugt im Bereich von 50°C bis 100°C durchgeführt wird, und/oder wobei das Trocknen in Schritt (e) vorzugsweise an Luft erfolgt.

11. Verfahren nach Anspruch 10, wobei die Nanopartikel eine Größe von 1 bis 20 nm, bevorzugt von 2 bis 10 nm und besonders bevorzugt von 5 bis 8 nm aufweisen, und/oder wobei die monodisperse Suspension bevorzugt eine wässrige Kieselsäuresuspension oder eine Suspension von Al₂O₃, TiO₂ oder ZrO₂ ist.

12. Verfahren nach einem oder mehreren der Ansprüche 10 oder 11, wobei die Gelbildung in Schritt (d) nach Ansäuern der monodispersen Suspension, bevorzugt auf einen pH-Wert von 1 bis 9, bevorzugter von 6 bis 8, und am bevorzugtesten von ungefähr 7, erfolgt, wobei das Ansäuern der Suspension vorzugsweise mit einer Mineralsäure, bevorzugt Salpetersäure oder Salzsäure, erfolgt.

13. Verfahren nach einem oder mehreren der Ansprüche 10 bis 12, wobei nach dem Trocknen des Festkörpers in Schritt (e) der Festkörper in einem weiteren Schritt calciniert wird, und/oder wobei nach dem Trocknen des Festkörpers dieser auf eine Temperatur von 300°C bis 600°C, bevorzugt 400°C bis 500°C, erhitzt wird, um organische Rückstände aus dem Festkörper zu entfernen und/oder um den porösen Festköper, der in Form einer gelartigen Matrix vorliegt, zu sintern, und/oder wobei nach dem Trocknen des Festkörpers dieser auf eine Temperatur von 700°C bis 1.200°C, bevorzugt 800°C bis 1.000°C, erhitzt wird, um den porösen Festköper, der in Form einer gelartigen Matrix vorliegt, in eine Glasmatrix umzuwandeln.

14. Beschichteter Körper, insbesondere ein Implantat, wobei der Körper mit einer Beschichtung beschichtet ist, die den porösen Festkörper gemäß einem oder mehreren der Ansprüche 1 bis 6 enthält.

15. Verwendung eines porösen Festkörpers gemäß einem oder mehreren der Ansprüche 1 bis 6, eines Granulats gemäß Anspruch 7 oder eines Formkörpers gemäß Anspruch 8 als Knochenersatzmaterial.

## Claims

1. Porous, inorganic solid body having a bimodal pore size distribution, comprising particles in an oxidic matrix and a content of antimicrobially acting metal cations, wherein
the particles are selected from calcium-containing and/or phosphorus-containing compounds, TiO₂, SiO₂, ZrO₂ and Al₂O₃, and wherein the particles have a particle size of 0.2-1000 µm,
the oxidic matrix contains one or more oxides of the elements Al, Ca, Mg, P, Si, Ti and Zr, and
the bimodal pore size distribution is configured in such a way that nanopores in the range of 1 to 100 nm are present in the oxidic matrix and the solid body further has macropores having a size of 50 to 1,000 µm.

2. Solid body according to claim 1, wherein the oxidic matrix contains one or more oxide, selected from Al₂O₃, TiO₂, SiO₂ and ZrO₂, and/or wherein the proportion of the oxidic matrix in the porous solid body is from 20 to 99 % by weight, preferably from 30 to 70 % by weight.

3. Solid body according to either claim 1 or claim 2, wherein the particles comprise a calcium phosphate, in particular hydroxylapatite or β-tricalcium phosphate, and/or wherein the particles have a particle size of 0.5-100 µm.

4. Solid body according to one or more of the preceding claims, wherein the metal cations are selected from ions of the metals copper, silver, chromium, zinc, cobalt, vanadium and mercury, and in particular are Cu²⁺, Cu⁺, Ag⁺, Cr³⁺, Cr⁶⁺, Zn²⁺, Co²⁺, V⁵⁺, Hg²⁺ and Hg⁺, and/or wherein the metal ions having an antimicrobial effect are present in the form of a salt, preferably as metal salts comprising organic anions, preferably copper acetate or silver acetate.

5. Solid body according to one or more of the preceding claims, wherein the bimodal pore size distribution is configured in such a way that nanopores in the range of 2 to 50 nm, more preferably 5 to 30 nm and most preferably 7 to 15 nm are present in the oxidic matrix, and the solid body further has macropores having a size of 200 to 500 µm and preferably 250 to 350 µm.

6. Solid body according to one or more of the preceding claims, wherein the oxidic matrix and/or the particles additionally contain further metal ions having a pharmaceutical effect, preferably Zn²⁺ and Ca²⁺ for promoting bone growth, and/or wherein the oxidic matrix of the solid body is converted in part or in whole into a glass matrix.

7. Granulate consisting of a solid body according to any of the preceding claims, wherein the granulate preferably has a particle size exceeding the macropore size by at least 5 times.

8. Shaped body or coating which, as well as a solid body according to one or more of claims 1 to 6, preferably in the form of a granulate according to claim 7, comprises a hardened liquid, the hardened liquid preferably being biodegradable and preferably comprising a polylactide or polysaccharide.

9. Method for producing a shaped body or a coating according to claim 8, wherein a solid body according to one or more of claims 1 to 6, preferably in the form of a granulate according to claim 7, is introduced into a hardenable liquid so as to produce a mixture, subsequently this mixture is brought into a mould or applied to a body, and the hardenable liquid is hardened in such a way that a shaped body or a coating results.

10. Method for producing a porous solid body according to one or more of claims 1 to 6, comprising the steps of:
(a) providing the sol thereof in the form of a largely monodisperse suspension of nanoparticles of at least one oxide of the elements Al, Ca, Mg, P, Si, Ti and Zr, wherein the nanoparticles have a particle size in the range of 0.1-50 nm;
(b) introducing particles into the suspension, the particles being selected from calcium-containing and/or phosphorus-containing compounds, TiO₂, SiO₂, ZrO₂ and Al₂O₃, the particles having a particle size of 0.2-1000 µm;
(c) introducing a salt containing metal cations, the metal cations having an antimicrobial effect;
(d) transferring the sol into a gel;
(e) drying the gel so as to generate a porous solid body;
wherein the drying in step (e) is preferably carried out at a temperature in the range of room temperature to 200 °C, preferably of 50 °C to 150 °C, and particularly preferably in the range of 50 °C to 100 °C, and/or wherein the drying in step (e) preferably takes place in air.

11. Method according to claim 10, wherein the nanoparticles have a size of 1 to 20 nm, preferably of 2 to 10 nm and particularly preferably of 5 to 8 nm, and/or wherein the monodisperse suspension is preferably an aqueous silica suspension or a suspension of Al₂O₃, TiO₂ or ZrO₂.

12. Method according to one or more of claims 10 or 11, wherein the gel formation in step (d) takes place after acidifying the monodisperse suspension, preferably to a pH of 1 to 9, more preferably of 6 to 8 and most preferably of approximately 7, the suspension preferably being acidified using a mineral acid, preferably nitric acid or hydrochloric acid.

13. Method according to one or more of claims 10 to 12, wherein after the solid body is dried in step (e) the solid body is calcinated in a further step, and/or wherein after the solid body is dried it is heated to a temperature of 300 °C to 600 °C, preferably 400 °C to 500 °C, so as to remove organic residues from the solid body and/or so as to sinter the porous solid body which is in the form of a gel-like matrix, and/or wherein after the solid body is dried it is heated to a temperature of 700 °C to 1,200 °C, preferably 800 °C to 1,000 °C, so as to convert the porous solid body which is in the form of a gel-like matrix into a glass matrix.

14. Coated body, in particular an implant, wherein the body is coated with a coating containing the porous solid body according to one or more of claims 1 to 6.

15. Use of a porous solid body according to one or more of claims 1 to 6, a granulate according to claim 7 or a shaped body according to claim 8 as bone replacement material.

## Revendications

1. Corps solide poreux inorganique muni d'une distribution de tailles de pores bimodale, comprenant des particules dans une matrice oxydante et une teneur en cations métalliques, qui agit de manière antimicrobienne, dans lequel
les particules sont choisies parmi des composés contenant du calcium et/ou du phosphore, du TiO₂, de la SiO₂, du ZrO₂ et de l'Al₂O₃, et dans lequel les particules présentent une taille particulaire de 0,2 à 1000 µm,
la matrice oxydante contient un ou plusieurs oxydes des éléments Al, Ca, Mg, P, Si, Ti et Zr, et
la distribution de la taille des pores bimodale est conçue de telle manière que des nanopores dans la plage de 1 à 100 nm se présentent dans la matrice oxydante et le corps solide présente en outre des macropores d'une taille de 50 à 1000 µm.

2. Corps solide selon la revendication 1, dans lequel la matrice oxydante contient un ou plusieurs oxydes, choisis parmi l'Al₂O₃, le TiO₂, la SiO₂ et le ZrO₂, et/ou dans lequel la proportion de matrice oxydante dans le corps solide poreux se situe de 20 à 99 % en poids, de préférence, de 30 à 70 % en poids.

3. Corps solide selon la revendication 1 ou 2, dans lequel les particules comprennent un phosphate de calcium, en particulier de l'hydroxy apatite de calcium ou du phosphate tricalcique β, et/ou dans lequel les particules présentent une taille particulaire de 0,5 à 100 µm.

4. Corps solide selon une ou plusieurs parmi les revendications précédentes, dans lequel les cations métalliques sont choisis parmi des ions des métaux cuivre, argent, chrome, zinc, cobalt, vanadium et mercure, et en particulier les ions sont Cu²⁺, Cu⁺, Ag⁺, Cr³⁺, Cr⁶⁺, Zn²⁺, Co²⁺, V⁵⁺, Hg²⁺ et Hg⁺, et/ou dans lequel les cations métalliques munis d'un effet antimicrobien se présentent sous la forme d'un sel, de préférence, sous la forme de sels métalliques avec des anions organiques, de préférence d'acétate de cuivre ou d'acétate d'argent.

5. Corps solide selon une ou plusieurs parmi les revendications précédentes, dans lequel la distribution des tailles des pores bimodale est conçue de telle manière que des nanopores dans la plage de 2 à 50 nm, de préférence, de 5 à 30 nm, et idéalement de 7 à 15 nm, sont présents dans la matrice oxydante, et le corps solide présente en outre des macropores munis d'une taille de 200 à 500 µm et, de préférence, de 250 à 350 µm.

6. Corps solide selon une ou plusieurs parmi les revendications précédentes, dans lequel la matrice oxydante, et/ou les particules contiennent en outre d'autres ions métalliques munis d'un effet pharmaceutique, de préférence, le Zn²⁺ et le Ca²⁺ pour la promotion de la croissance des os, et/ou dans lequel la matrice oxydante du corps solide est transformée partiellement ou totalement en une matrice vitreuse.

7. Granulat à base d'un corps solide selon l'une des revendications précédentes, le granulat possédant de préférence une taille particulaire qui dépasse d'au moins 5 fois la taille des macropores.

8. Corps moulé ou revêtement qui contiennent un liquide figé à côté d'un corps solide selon une ou plusieurs parmi les revendications 1 à 6, de préférence sous la forme d'un granulat selon la revendication 7, dans lequel le liquide figé est de préférence biodégradable et comprend de préférence un polylactide ou un polysaccharide.

9. Procédé de fabrication d'un corps moulé ou d'un revêtement selon la revendication 8, dans lequel un liquide pouvant être figé est incorporé dans un corps solide selon une ou plusieurs parmi les revendications 1 à 6, de préférence, sous la forme d'un granulat selon la revendication 7, afin de produire un mélange, ensuite, ce mélange est transformé dans une forme, respectivement, est rapporté sur un corps, et le liquide pouvant être figé est figé de sorte qu'il en résulte un corps moulé, respectivement, un revêtement.

10. Procédé de fabrication d'un corps solide poreux selon une ou plusieurs parmi les revendications 1 à 6 comportant les étapes :
(a) de préparation d'un sol sous la forme d'une suspension dans la mesure du possible monodisperse à base de nanoparticules d'au moins un oxyde des éléments Al, Ca, Mg, P, Si, Ti et Zr, les nanoparticules présentant une taille particulaire dans la plage de 0,1 à 50 nm ;
(b) d'incorporation de particules dans la suspension, les particules étant choisies parmi des composés contenant du calcium et/ou du phosphore, du TiO₂, de la SiO₂, du ZrO₂ et de l'Al₂O₃, et les particules présentant une taille particulaire de 0,2 à 1000 µm ;
(c) d'incorporation d'un sel contenant des cations métalliques, les cations métalliques possédant un effet antimicrobien ;
(d) de transformation du sol en un gel ;
(e) de séchage du gel afin de créer un corps solide poreux ;
le séchage dans l'étape (e) étant effectué de préférence à une température dans la plage de la température ambiante jusqu'à 200 °C, de préférence de 50 °C à 150 °C, et de manière particulièrement préférée, dans la plage de 50 °C à 100 °C, et/ou dans lequel le séchage dans l'étape (e) ayant lieu de préférence à l'air.

11. Procédé selon la revendication 10, dans lequel les nanoparticules présentent une taille de 1 à 20 nm, de préférence de 2 à 10 nm et idéalement de 5 à 8 nm, et/ou la suspension mono disperse est de préférence une suspension aqueuse d'acide silicique ou une suspension d'Al₂O₃, de TiO₂ ou de ZrO₂.

12. Procédé selon une ou plusieurs parmi les revendications 10 ou 11, dans lequel la formation du gel dans l'étape (d) a lieu après une acidification de la suspension monodisperse, de préférence, à une valeur de pH de 1 à 9, de manière préférée de 6 à 8, et idéalement d'environ 7, dans lequel l'acidification de la suspension a lieu de préférence avec un acide minéral, de préférence de l'acide nitrique ou de l'acide chlorhydrique.

13. Procédé selon une ou plusieurs parmi les revendications 10 à 12, dans lequel, après le séchage dans l'étape (e), le corps solide est calciné dans une étape supplémentaire, et/ou, dans lequel, après le séchage du corps solide, celui-ci est chauffé à une température de 300 °C à 600 °C, de préférence, de 400 °C à 500 °C, afin d'enlever les résidus organiques du corps solide et/ou fritter le corps solide poreux qui se présente sous la forme d'une matrice de type gel, et/ou dans lequel, après le séchage du corps solide, celui-ci est chauffé à une température de 700 °C à 1200 °C, de préférence, de 800 °C à 1000 °C, afin de transformer le corps solide poreux qui est présent sous la forme d'une matrice de type gel en une matrice vitreuse.

14. Corps revêtu, en particulier, implant, le corps étant revêtu d'un revêtement qui contient le corps solide poreux selon une ou plusieurs parmi les revendications 1 à 6.

15. Utilisation d'un corps solide poreux selon une ou plusieurs parmi les revendications 1 à 6, d'un granulat selon la revendication 7 ou d'un corps moulé selon la revendication 8 en tant que matière de remplacement d'os.
